Europäisches Patentamt

European Patent Office

Office européen des brevets

(19)

(11) Veröffentlichungsnummer: **0 080 572**
**B1**

(12) **EUROPÄISCHE PATENTSCHRIFT**

(45) Veröffentlichungstag der Patentschrift:
05.12.84

(21) Anmeldenummer: 82108905.9

(22) Anmeldetag: 25.09.82

(51) Int. Cl.³: **C 07 C 45/82**, C 07 C 47/04

(54) Verfahren zur Herstellung reinen Methylals.

(30) Priorität: 28.11.81 DE 3147320

(43) Veröffentlichungstag der Anmeldung:
08.06.83 Patentblatt 83/23

(45) Bekanntmachung des Hinweises auf die Patenterteilung:
05.12.84 Patentblatt 84/49

(84) Benannte Vertragsstaaten:
AT BE CH DE FR GB IT LI NL SE

(56) Entgegenhaltungen:
DE - B - 1 290 534
US - A - 3 077 441

(73) Patentinhaber: CHEMISCHE WERKE HÜLS AG, - RSP Patente / PB 15 - Postfach 13 20, D-4370 Marl 1 (DE)

(72) Erfinder: Müller, Wolfgang Hans Eduard, Dr., Bitterfelder Strasse 9a, D-4370 Marl (DE)
Erfinder: Kaufhold, Manfred, Dr., Jasminweg 20, D-4370 Marl (DE)

ACTORUM AG

## Beschreibung

Die Erfindung betrifft ein Verfahren zur Gewinnung von reinem Methylal aus Gemischen von Methylal und Methanol, die gegebenenfalls noch weitere Stoffe wie Wasser, Formaldehyd, Methylformiat und andere enthalten können, insbesondere ein Verfahren zur Gewinnung von reinem Methylal aus Mischungen, wie sie bei der Synthese des Methylals aus Formalinlösung und Methanol erhalten werden.

Die Herstellung eines ca. 8%igen Methanol enthaltenden Methylals ist technisch mit Eisen-(III)chlorid als Katalysator leicht durchführbar und bedarf keiner besonderen technischen Einrichtungen (siehe Houben-Weyl „Methoden der organ. Chemie", Bd. VI/3, Sauerstoffverb. 1, Teil 3 [1965], S. 207). Vorzugsweise setzt man anstelle von Eisen(III)chlorid Eisen(III)sulfat ein, das möglichst wenig Chlor enthält, um die Bildung von chlorhaltigen, giftigen Nebenprodukten zu vermeiden.

Die Gewinnung von reinem Methylal aus diesen Mischungen durch einfache Rektifikation gelingt wegen des Vorhandenseins eines Azeotrops nicht. Nach Literaturangaben werden durch einfache Rektifikation Gemische mit z.B. 8 Gew.-% Methanol erhalten. Für die Abtrennung dieser durch einfache Rektifikation nicht abtrennbaren Methanolmengen werden verschiedene physikalische oder chemische Verfahren empfohlen.

Volkov und Ivanov („Vysokomolekul. Soedin", 8 [8], 1459-61 [1966]) und Vinokurov („Nauch. Doklady Vysskei Shkoly. Lesoinzhener. Delo.", 1958, Nr. 4, 193-195) reinigen das Methylal durch chemische Umsetzung des anwesenden Methanols mit metallischem Natrium.

Das Methanol kann auch durch Ausschütteln mit konzentrierter wässeriger Chlorkalziumlösung und anschliessendes Trocknen des Methylals entfernt werden (Ullmanns „Encyklopädie der technischen Chemie", 3. Aufl., Bd. 3, S. 15). Weiter werden für die Trennung von Methanol und Methylal Extraktivrektifikationen mit verschiedenen Hilfsstoffen angegeben. Als Hilfsstoffe werden genannt: Wasser (DE-PS Nr. 1002305) (US-PS Nr. 2545889), wässerige alkalische Lösungen (US-PS Nr. 2990340) (Vinokurov „Gidroliz. i Lesokhim. Prom.", 12, Nr. 5, S. 4 bis 5 [1959]), Ethylenglykol (DE-PS Nr. 1127339), Paraformaldehyd (Vinokurov „Gidroliz. i Lesokhim. Prom.", 12, Nr. 5, S. 4 bis 6 [1959]) sowie Dimethylformamid (DE-PS Nr. 1172677). Darüber hinaus wird die chemische Umsetzung des Methanols mit einem Aldehydüberschuss zum Acetal beschrieben (SU-PS Nr. 121443).

Vinokurov („Izvest. Vysshikh Ucheb. Zavedenii, Lesnoi Zhur.", 2, Nr. 2, 155-159 [1959]) vergleicht drei verschiedene Methoden zur Trennung von Methanol und Methylal: Umsetzung des Methanols mit Paraformaldehyd, Behandlung der azeotropen Mischung von Methanol und Methylal mit Natriumhydroxid und Destillation sowie Rektifikation in Gegenwart eines Überschusses von Formalinlösung.

Alle diese beschriebenen Verfahren benötigen den Einsatz weiterer Stoffe. Sie sind sehr aufwendig und ergeben z. T. geringere Ausbeuten.

Damit stellt sich die Aufgabe, ein Trennverfahren für unreines Methylal zu entwickeln, das praktisch quantitative Ausbeuten an Methylal ermöglicht und keine Methanolverluste bringt sowie keine fremden Stoffe einsetzt. Es soll eine hohe Reinheit des gewonnenen Methylals ohne weitere Reinigungsschritte erreicht werden. Dieses neue Reinigungsverfahren für Methylal soll zudem auch einfach und wirtschaftlich sein.

Diese Aufgabe wird erfindungsgemäss entsprechend den Angaben der Ansprüche gelöst.

Man rektifiziert das Methylal, Methanol und gegebenenfalls noch weitere Stoffe enthaltende Rohprodukte in zwei unter unterschiedlichem Druck betriebenen Rektifikationskolonnen. In der ersten unter einem Druck von 1 bis 22 bar absolut betriebenen Kolonne destilliert man das azeotrope Gemisch Methylal/Methanol, das gegebenenfalls noch die leichtersiedenden Stoffe wie Methylformiat enthält, von dem Überschuss an Methanol und gegebenenfalls noch höhersiedenden Stoffe wie z.B. Wasser ab. Das Destillat dieser ersten Rektifikationskolonne trennt man in einer zweiten unter höherem Druck von 9 bis 30 bar absolut betriebenen Rektifikationskolonne in einen methanolhaltigen Stoffstrom, der grössere Anteile an Methylal enthält, und reines Methylal als Sumpfprodukt. Den methanolhaltigen Stoffstrom kann man der zweiten Kolonne am Kolonnenkopf entnehmen. Man kann den methanolhaltigen Stoffstrom auch als Seitenstrom aus dem Verstärkungsteil der zweiten Kolonne entnehmen und am Kolonnenkopf die im Rohstoff vorhandenen leichtersiedenden Stoffe wie Methylformiat in hoher Konzentration abziehen.

Bei dem erfindungsgemässen Verfahren erhält man als Sumpfprodukt der zweiten Rektifikation ein reines Methylal, das insbesondere weder Methanol noch Methylformiat noch Wasser enthält. Das Methanol und Methylal enthaltende Destillat der zweiten Rektifikation gibt man zur ersten Rektifikationskolonne zurück.

Wesentlich für das erfindungsgemässe Verfahren ist die Rektifikation in zwei Rektifizierkolonnen, wobei man die zweite Rektifikation unter einem höheren Druck durchführt. Vorzugsweise betreibt man die zweite Rektifikation bei einem um mindestens 8 bar höheren Druck.

Bei Anwesenheit von Leichtsiedern, wie z.B. Methylformiat, kann man einen Teilstrom des Methylal enthaltenden Destillats der zweiten Rektifikationskolonne verwerfen. Dies bedeutet jedoch eine Ausbeuteminderung an Methylal. Man trennt daher vorzugsweise das Destillat der zweiten Kolonne in einer weiteren Rektifikation in nahezu reine Leichtsieder, die aus dem Verfahren ausgeschleust werden, und in ein nahezu leichtsiederfreies Gemisch von Methanol und Methylal, das der ersten Rektifikationskolonne zugeführt wird. Besonders vorteilhaft ist die Einbeziehung dieser Leichtsiederabtrennung in die zweite Rektifikation, wobei ein leichtsiederarmer methanol- und methylalhaltiger Seitenstrom aus dem Verstärkungsteil dieser Rektifikationskolonne entnommen wird, der zur ersten Kolonne zurückgeführt wird und ein methanol- und methylalarmes Destillat am Kolonnenkopf entnommen wird, während im Sumpf dieser Rektifikationskolonne reines Methylal anfällt. Das Sumpfprodukt der ersten Rektifikation, das insbesondere aus Methanol und Wasser besteht, kann in bekannter Weise, z.B. durch Rektifikation in seine Bestandteile, getrennt werden. Das erfindungsgemässe Verfahren führt man vorzugsweise kontinuierlich durch.

Der Vorteil des erfindungsgemässen Verfahrens besteht gegenüber dem Stand der Technik in der besonders einfachen, leicht zu automatisierenden Technik, da nur einfache Rektifikationen ohne jeden Fremdstoffzusatz angewendet werden. Das Verfahren liefert ohne Verluste an Methanol eine quantitative Ausbeute an Methylal. Das Verfahren ist flexibel, da in einer Anlage leicht Rohstoffe unterschiedlicher Zusammensetzung mit und ohne weitere Stoffe, wie z.B. Methylformiat, verarbeitet werden können. Das Verfahren ist wirtschaftlich und kann gegebenenfalls auch für sehr grosse Kapazitäten angewendet werden.

Das Verfahren ist umweltfreundlich, da keine weiteren Stoffe benötigt werden, die zu Belastungen der Umwelt oder zu Verunreinigungen des Produktes führen können.

*Beispiel 1:*

Ein Gemisch aus 50 Gew.-Teilen Methanol und 50 Gew.-Teilen Methylal führt man kontinuierlich flüssig mit Siedetemperatur dem 15. praktischen Boden (von unten gezählt) einer im ganzen 35 praktische Böden aufweisenden Rektifikationskolonne zu. Auf den 16. Boden, von unten gezählt, leitet man das Kopfprodukt der zweiten Destillationskolonne ebenfalls flüssig mit Siedetemperatur. Die erste Rektifikationskolonne betreibt man unter Normaldruck. Am Kolonnenkopf nimmt man mit einem Rücklaufverhältnis von 2 ein Produkt mit 95,0 Gew.-% Methylal ab, während man im Kolonnensumpf ein Produkt, Methanol, mit etwa 10 Gew.-ppm Methylal erhält. Das am Kopf anfallende Produkt mit 95 Gew.-% Methylal leitet man in die Mitte, zwischen Kopf und Sumpf, einer zweiten, mit Füllkörpern entsprechend einer theoretischen Stufenzahl von 35 theoretischen Stufen gefüllten Kolonne. Diese Kolonne betreibt man unter einem Druck von 10 bar absolut am Kolonnenkopf. Am Kolonnenkopf entnimmt man mit einem Rücklaufverhältnis von 0,9 ein Destillat mit 15,9 Gew.-% Methanol, Rest Methylal, das man zur ersten Kolonne zurückführt. Als Kolonnensumpf dieser zweiten Rektifikation erhält man ein reines Methylal, das nur 10 Gew.-ppm Methanol enthält.

*Beispiel 2:*

Die Versuchsdurchführung erfolgt wie in Beispiel 1 beschrieben. Die zweite Kolonne ist jedoch um 50% höher, wobei der Zulauf in 1/3 Höhe der Kolonne und die Abnahme eines flüssigen Seitenstromes in 2/3 Höhe der Kolonne erfolgt. Am Kopf der Kolonne wird nichts abgenommen, da Leichtsieder wie im Beispiel 1 im Rohstoff nicht anwesend sind. Die Menge des Seitenstroms, die in 2/3 Kolonnenhöhe flüssig abgenommen und zur ersten Rektifikationskolonne zurückgeführt wird, entspricht 52,6% der an dieser Stelle durch die Kolonne fliessenden Flüssigkeitsmenge.

Man erhält die gleichen Ergebnisse wie in Beispiel 1.

*Beispiel 3:*

Die Versuchsdurchführung erfolgt wie in Beispiel 2, jedoch enthält der Zulauf zur ersten Kolonne jetzt 50 Gew.-Teile Methylal, 40 Gew.-Teile Methanol und 1 Gew.-Teil Methylformiat. Vom Kopf der ersten Kolonne nimmt man mit Rücklaufverhältnis 2 76% Destillat, bezogen auf den oben genannten Zulauf, ab und leitet es der zweiten Kolonne zu. Von der zweiten Kolonne führt man 25% Seitenstrom, bezogen auf den Zulauf zur ersten Kolonne, zur ersten Kolonne zurück. Als Sumpfprodukt der ersten Kolonne fallen 49% (Methanol) an, bezogen auf den Zulauf, mit nur 0,1% Methylal und frei von Methylformiat. Am Kopf der zweiten unter 10 bar Druck betriebenen Kolonne nimmt man ca. 1%, bezogen auf den Zulauf zur ersten Kolonne, mit einem Rücklaufverhältnis von 50 ab. 25%, bezogen auf Zulauf zur ersten Kolonne, leitet man als Seitenstrom zur ersten Kolonne zurück. Im Sumpf der zweiten Kolonne fallen ca. 50% Methylal, bezogen auf den Zulauf zur ersten Kolonne, mit weniger als 10 Gew.-ppm Methanol und frei von Methylformiat an.

*Beispiel 4:*

Die Versuchsdurchführung erfolgt nach den Angaben des Beispiels 3, jedoch verwendet man einen Rohstoff, der 20 Gew.-% Wasser, 40 Gew.-% Methylal, 39,2 Gew.-% Methanol und 0,8 Gew.-% Methylformiat enthält. Bei Abnahme von ca. 66% Destillat mit Rücklaufverhältnis 3 aus der ersten Kolonne, die der zweiten Kolonne zugeleitet werden, und einer Rückführung des Seitenstromes von 25%, bezogen auf den Zulauf zur ersten Kolonne, von der zweiten Kolonne zur ersten Kolonne, fällt in der ersten Kolonne ein Sumpfprodukt von 59%, bezogen auf den Zulauf, an mit einer Zusammensetzung von ca. 33,9% Wasser, 66,0% Methanol und 0,1% Methylal.

Die Fahrweise und die Konzentration der Kolonne 2 ist die gleiche wie in Beispiel 3. Der Wassergehalt des Sumpfproduktes, reines Methylal, liegt unter 0,1 Gew.-%.

**Patentansprüche**

1. Verfahren zur Gewinnung von reinem Methylal durch Trennung von Methanol/Methylal-Gemischen, die gegebenenfalls noch weitere Stoffe enthalten können, dadurch gekennzeichnet, dass man das Gemisch in einer Rektifiziereinrichtung aus zwei Rektifizierkolonnen trennt, wobei man das zu trennende Gemisch in einer ersten Rektifikation bei einem Druck von 1 bis 22 bar absolut in ein methylalreiches, methanolarmes Destillat und ein praktisch methylalfreies, methanolreiches Sumpfprodukt zerlegt und das methylalreiche Destillat der ersten Rektifikation in einer zweiten unter höherem Druck von 9 bis 30 bar absolut betriebenen Rektifikation in einen Methanol und Methylal enthaltenden Stoffstrom, den man zur ersten Rektifikation zurückführt, und reines Methylal als Sumpfprodukt trennt.

2. Verfahren nach Anspruch 1, dadurch gekennzeichnet, dass man die zweite Rektifikation bei einem um mindestens 8 bar höheren Druck als die erste Rektifikation ausführt.

3. Verfahren nach einem der beiden Ansprüche 1 oder 2, dadurch gekennzeichnet, dass man den Methanol und Methylal enthaltenden Stoffstrom der zweiten unter höherem Druck betriebenen Rektifikation am Kolonnenkopf abnimmt.

4. Verfahren nach einem der beiden Ansprüche 1 oder 2, dadurch gekennzeichnet, dass man den Methanol und Methylal enthaltenden Stoffstrom der zweiten unter höherem Druck betriebenen Rektifikation als Seitenstrom aus dem Verstärkungsteil der Kolonne entnimmt und am Kolonnenkopf die im Rohstoff vorhandenen leichtersiedenden Stoffe in hoher Konzentration entnimmt.

## Claims

1. A process for obtaining pure methylal by separating a methanol/methylal mixture which optionally also contains one or more other substances, characterised in that the mixture is separated in a rectifying unit comprising two rectifying columns, the mixture to be separated being separated into a methylal-rich, low-methanol distillate and a virtually methylal-free, methanol-rich bottom product, in a first rectification operated at a pressure of 1 to 22 bar absolute, and the methylal-rich distillate from the first rectification being separated, in a second rectification operated under a higher pressure of 9 to 30 bar absolute, into a stream of material containing methanol and methylal, which is recycled to the first rectification, and pure methylal as the bottom product.

2. A process according to Claim 1, characterised in that the second rectification is carried out at a pressure which is at least 8 bar higher than that of the first rectification.

3. A process according to one of Claims 1 or 2, characterised in that the stream of material containing methanol and methylal from the second rectification, which is carried out under higher pressure, is taken off at the top of the column.

4. A process according to one of Claims 1 or 2, characterised in that the stream of material containing methanol and methylal from the second rectification, which is carried out under higher pressure, is taken as a side stream from the enrichment section of the column, while at the top of the column the lighter-boiling materials present in the raw material are taken off at a high concentration.

## Revendications

1. Procédé d'obtention de méthylal à l'état pur par séparation de mélanges de méthanol et de méthylal qui peuvent éventuellement renfermer encore d'autres substances, caractérisé par le fait qu'on sépare le mélange dans une installation de rectification constituée par deux colonnes de rectification, tandis qu'on décompose le mélange à séparer, lors d'une première rectification effectuée sous une pression de 1 à 22 bar absolus, en un distillat riche en méthylal et pauvre en méthanol et en un produit de queue riche en méthanol et pratiquement exempt de méthylal, et qu'on décompose, sous une pression plus élevée de 9 à 30 bar absolus, le distillat riche en méthylal, qui provient de la première rectification, en un courant de substances renfermant du méthanol et du méthylal, que l'on renvoie à la première rectification, et en méthylal pur en tant que produit de queue.

2. Procédé selon la revendication 1, caractérisé par le fait qu'on effectue la deuxième rectification sous une pression supérieure de 8 bar au moins à la pression sous laquelle est effectuée la première rectification.

3. Procédé selon l'une des revendications 1 ou 2, caractérisé par le fait que l'on soutire à la tête de la colonne le courant de substances renfermant du méthanol et du méthylal, qui provient de la seconde rectification effectuée sous une pression plus élevée.

4. Procédé selon l'une des revendications 1 ou 2, caractérisé par le fait que l'on prélève comme courant latéral, de la partie de renforcement de la colonne, le courant de substances, renfermant du méthanol et du méthylal, qui provient de la deuxième rectification effectuée sous pression plus élevée et que l'on prélève à la tête de la colonne, à haute concentration, les substances à plus bas point d'ébullition qui sont présentes dans le produit brut.